(19) Europäisches Patentamt / European Patent Office / Office européen des brevets

(11) **EP 2 180 872 B1**

(12) **EUROPEAN PATENT SPECIFICATION**

(45) Date of publication and mention
of the grant of the patent:
**01.01.2020 Bulletin 2020/01**

(21) Application number: **08788237.9**

(22) Date of filing: **29.07.2008**

(51) Int Cl.:
*A61K 6/00* (2006.01)  *C08B 37/00* (2006.01)
*C08L 5/00* (2006.01)  *C09J 105/00* (2006.01)
*A61L 15/58* (2006.01)

(86) International application number:
**PCT/GB2008/002585**

(87) International publication number:
**WO 2009/016362 (05.02.2009 Gazette 2009/06)**

(54) **COMPOSITIONS COMPRISING POLYSACCHARIDE GUMS**

POLYSACCHARID-GUMMIS UMFASSENDE ZUSAMMENSETZUNGEN

COMPOSITIONS COMPRENANT DES GOMMES POLYSACCHARIDIQUES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **30.07.2007 GB 0714775**

(43) Date of publication of application:
**05.05.2010 Bulletin 2010/18**

(73) Proprietors:
• **SAN-EI GEN F.F.I., INC.**
**Toyonaka-shi,**
**Osaka 561-8588 (JP)**
• **RECKITT & COLMAN (OVERSEAS) HEALTH
LIMITED**
**Slough, Berkshire SL1 3UH (GB)**

(72) Inventors:
• **AL-ASSAF, Saphwan**
**North Wales LL11 2AW (GB)**

• **DICKSON, Paul, Andrew**
**Hull HU8 7DS (GB)**
• **PHILLIPS, Glyn, O.**
**North Wales LL11 2AW (GB)**
• **THOMPSON, Carl**
**Hull HU8 7DS (GB)**
• **TORRES, Jesus, Cirre**
**North Wales LL11 2AW (GB)**

(74) Representative: **Cawdell, Karen Teresa et al**
**Reckitt Benckiser
Corporate Services Limited
Legal Department - Patents Group
Dansom Lane
Hull HU8 7DS (GB)**

(56) References cited:
**DE-A1- 1 909 209    US-A- 3 833 518
US-A- 4 356 819    US-A- 4 474 902**

**Description**

The present invention relates to the use of polysaccharide gums in adhesives, especially in denture adhesives. It further relates to modified forms of naturally occurring polysaccharide gums, including ghatti, karaya and kerensis gums, which are especially useful in this respect, either alone or blended with unmodified gums. The present invention further relates to the methods of preparation of such modified gums, and specifically to the control of hydrogel content of the modified gums, which is key for their performance in the aforementioned adhesive compositions.

There are several known naturally occurring polysaccharide gums, of which gum ghatti, gum karaya and gum kerensis are examples.

Gum ghatti, also known as Indian gum, is the dried exudate of *Anogeissus latifolia,* a large tree found abundantly in the dry deciduous forests of India. Gum ghatti was originally developed around 1900 as a substitute for gum arabic. However, several studies have demonstrated that the raw form of the gum is not currently suitable for certain applications due to the variation in solubility and viscosity of the raw material. Consequently, gum ghatti has never been established as major tree gum for industrial use.

The viscosity of natural gum ghatti has been shown to vary from 30 to 400cP for 5% solutions. Collected nodules are not entirely soluble in cold water; however, breaking up and heating the gum at 90°C has been shown to improve the solubility. Although largely water soluble, individual samples may contain varying proportions of insoluble gel but are not chemically different to the soluble component.

The unpredictable physical and chemical properties of gum ghatti have prevented its commercial application. Natural gum exudates, including gum ghatti, have adhesive qualities which are dependent on their natural viscoelasticity. These adhesive qualities can be variable, due to the unpredictability of the molecular properties of the gum. This is common for natural exudates whose properties often dependent on the geography, climate and soil origin of the tree from which the gum is obtained.

One of the problems associated with gum ghatti is the variation in viscosity from batch to batch. This variation is often due to the difference in insoluble hydrogel content. As noted, this variation is to be expected for natural material and has been shown with, for example, gum arabic, whose properties and molecular parameters are dependent upon the age and location of the acacia trees from which it is derived. This variation also leads to unpredictable functional applicability.

Gum karaya is obtained from *Sterculia urens* R and other *Sterculia* spp. (Sterculiaceae, Malvales) and has been used as a denture adhesive/fixative agent in its neat (powder) form. Similarly to gum ghatti, gum karaya exhibits a natural variation of the proportion of soluble and insoluble fraction depending on the location of the tree or season when the gum is extracted. Gum kerensis is a gum exudate obtained from acacia tree (otherwise known as *A.senegal var. karensis.*) by tapping.

Hydrogels are three-dimensional cross-linked network structures in a polymeric system which are insoluble in water but which are able to absorb many times their weight of water. Hydrogels also tend to respond to the surrounding environment and to stimuli such as for example temperature, pH and the presence of electrolytes. The procedure of producing hydrogel (gel) often involves chemical reagents to cross-link the polymer chains to produce the three dimensional network structures. Hydrogels have been developed from many synthetic or natural polymers (hydrocolloids) as well as mixtures of the two. Due to their high water absorption capacity, hydrogels have been used in wound dressing, drug delivery, agriculture, sanitary pads as well as transdermal systems, denture materials, implants, injectable polymeric systems, ophthalmic applications and hybrid-type organs (encapsulated living cells) . Hyaluronan and alginate are among the most frequently utilised natural polysaccharides (hydrocolloids) hydrogels. Cellulose and its derivatives (carboxymethyl cellulose, hydroxypropyl methyl cellulose) also play an important role in the realisation of systems for the controlled release of drugs.

It is, therefore, important to be able to prepare hydrogels with controlled hydrodynamic properties, swelling, water absorption and hydration rate.

It is therefore the aim of the present invention to eliminate the natural variability that exists in samples of naturally occurring polysaccharide gums and produce products with reproducible viscoelasticity.

US-A-3833518 describes an anhydrous denture adhesive containing a mixture of a cationic polymeric material and a natural or synthetic anionic gum material as the adhesive ingredient.

DE-A-1909209 describes a dental adhesive comprising a synthetic or natural gum, vaseline and natural wood meal.

US-A-4356819 describes an article of manufacture with adhesive properties comprising a gelatinous adhesive composition dispersed in a plastics matrix. The gelatinous adhesive composition is the resulting product of heating together in the absence of solvent at least one polyhydric alcohol and at least one material selected from the group consisting of gelatin, naturally occurring high molecular weight polysaccharide gums, and resins which are a copolymer of a vinyl ether and a second component selected from organic acid anhydrides and their corresponding free acids.

US-A-4474902 describes a denture adhesive containing a karaya gum adhesive component in a hydrophilic vehicle comprising certain polyethylene glycols.

Surprisingly it has been found that thermal modification of naturally occurring gums, in the solid state, leads to an unexpected improvement in their hydrodynamic properties, their ability to form stable hydrogels and their consequent performance in adhesive materials, with much greater reliability and consistency than had previously been the case with unmodified materials.

The present invention concerns the use of a range of natural gums which contain proportions of soluble and insoluble fractions which can be used alone or blends thereof in a denture adhesive, wherein the gum may comprise alone or as part of a blend of one or more modified forms of gum that can be produced by heating natural gum in the dry solid state under specific conditions of temperature and atmospheric pressure to control the ratio of insoluble to soluble fraction. The modified gums have physical properties that are suitable for denture adhesive and which are not always solely present in the naturally occurring gum; in particular, the modified gum products have higher viscoelastic properties, increased swelling capacities and slow hydration compared with the naturally occurring gums. Such modification of the natural materials using this defined process allows the material to be adapted to fit the requirements of an effective denture adhesive, i.e. viscoelastic, an elastic dominance over a wide frequency range and a high dynamic viscosity.

According to a first aspect of the present invention, there is provided a process for the manufacture of a modified form of a polysaccharide gum which comprises the step of heating the natural gum, in the solid state, at atmospheric pressure at a temperature of between 100 to 130°C for at least 12 hours,
wherein the modified gum has a gel fraction of between 10 and 80 wt% and a hydration value of between 60 and 10 wt%.

Preferably there is provided a process of the first aspect, which comprises heating for at least 24 hours, more preferably at least 3 days, further preferably at least 4 days, most preferably at least 6 days, especially at least 7 days, especially at least 14 days.

Preferably there is provided a process of the first aspect, wherein the polysaccharide gum is, or is derived from, one or more of natural karaya, ghatti or kerensis gums.

According to a second aspect of the present invention there is provided the use of a modified polysaccharide gum produced according to a process of the first aspect, in a denture adhesive.

Preferably the gel fraction of the modified gum produced by the process of the first aspect of the present invention is > 20 wt%, most preferably > 30 wt%.

Preferably the hydration value of the modified gum produced by the process of the first aspect of the present invention is < 40 wt%, especially < 20 wt%.

Gel fraction and hydration value are each measured by the experimental technique set out in the Examples section below. Hydration value is defined in Table 1 as the solubility or the % of the soluble fraction (based on the total weight of the gum).

Preferably the gum modified according to the first aspect of the present invention is obtained after heating a sample of the gum in a dry solid state at 110°C and atmospheric pressure for at least 12 hours, preferably at least 24 hours, more preferably at least 3 days, further preferably at least 4 days, most preferably at least 6 days, especially at least 7 days. Heating time can be greatly reduced if the material is heated in spray dried form or changing the ambient conditions to a controlled pressure and humidity.

The gel content and hydration value of the gum(s) will influence their ability to be dissolved in water or other solvent systems, and will determine the properties of such solutions or suspensions, in particular the viscosity of such solutions or suspensions. In a preferred embodiment of the first aspect of the present invention, there is provided a modified polysaccharide gum produced by the process which, when suspended or dissolved as a 10wt% dispersion in water, has a dynamic viscosity of at least 10 Pa.s at an oscillation frequency of 0.1Hz.

In a preferred embodiment, the adhesive according to the first aspect of the present invention may comprise a single gum or a blend of two or more gums. The two or more gums may be from the same natural source (e.g. both ghatti gums) or from different sources (e.g. ghatti and karaya gums) and may also be a mixture of naturally occurring gums of natural and modified gums.

Preferably the denture adhesive specified in the second aspect of the present invention comprises a blend of at least two gums wherein the total hydrogel content is in the range of 10 - 80% and the molecular weight of the soluble fraction is > 1.0 million.

Preferably the denture adhesive according to the first aspect of the invention is in the form of an ointment, cream or gel.

Preferably the denture adhesive specified in the second aspect of the invention comprises one or more adjuvants selected from but not restricted to oils, waxes, gums, further adhesive substances, fragrances and dyes.

In a preferred embodiment, the denture adhesive composition specified in the second aspect of the invention comprises:

- 0.1 - 60 wt% of at least one modified polysaccharide gum according to the first aspect of the invention;

- 0.1 - 90 wt% of a base for the cream, ointment or gel; and,
- 0.1 - 50 wt% of at least one further adhesive substance

based on the overall composition.

Preferably the combined amounts in wt% of the at least one modified polysaccharide gum and the at least one further adhesive substance is less than 60 wt%, based on the overall composition.

Preferably the oil/wax is selected from the group consisting of paraffin oil, sunflower oil, soy oil, petroleum jelly (Vaseline), glycerine, sorbitol, soya bean, vegetable wax (which may include blends of oils and waxes such as, but not limited to, castor seed oil, hydrogenated castor oil and carnuba wax) and hydrophobic oleogels; more preferably the oil/wax is selected from petrolatum, sunflower oil or paraffin oil or mixtures thereof.

Preferably, the denture adhesive specified in the second aspect of the invention may further comprise one or more additional active adhesive substances, selected from the group consisting of sodium carboxymethylcellulose, sodium alginate, copolymer salts of methyl vinyl ether/maleic anhydride, polyvinyl acetate, polyvinyl pyrrolidone, hydroxyethyl cellulose, polyoxymethylene, polyacrylamides, polyethylene oxides and mixtures thereof.

Preferably the denture adhesive specified in the second aspect of the invention comprises an additional active adhesive substance which is a derivative of cellulose. Preferred is carboxymethylcellulose (CMC).

Further preferred as additional active adhesive substances are copolymer salts of methyl vinyl ether/maleic anhydride. In one particularly preferred embodiment of the present invention, a mixture of sodium carboxymethylcellulose and copolymer salts of methyl vinyl ether/maleic anhydride are added as additional adhesive substances.

It is preferred that the denture adhesive specified in the second aspect of the present invention has a viscosity of between 5 and 50 Pa.s at $10s^{-1}$, measured using a cone and plate Rheometer at 25°C and atmospheric pressure; and, a tube extrusion force of <16kg, measured using a texture analyser at 25°C and atmospheric pressure.

The preferred heating time selected for the process of the first aspect will depend on the ambient conditions and on the method used to produce the initial unmodified solid gum.

A denture adhesive specified in the second aspect of the invention may be produced comprising the steps of:

- weigh the oil/gel phase components
- add the solid components
- mix until homogeneous.

Preferably the weighing of the oil/gel phase components is carried out at a temperature above ambient temperature, more preferably at 70 °C or above, and the resultant mixture is allowed to cool before the solid phase components are added, preferably to below 50 °C, more preferably to below 40 °C.

## Examples

The invention will now be further described by way of the following experimental data with reference to the accompanying drawings in which:

**Figure 1** illustrates the dynamic viscosity values for control and modified (processed) gum ghatti samples according to the present invention as a function of oscillation frequency. The recorded measurements were performed at 25°C on 10% w/w (based on loss on drying) in water containing 0.005% sodium azide ($NaN_3$)

**Figure 2** illustrates the viscoelastic parameter, G' (open symbols) and G" (closed symbols), of 10% w/w control and modified ghatti dispersion as a function of oscillation frequency.

**Figure 3** illustrates the % gel fraction and % solubility/hydration values as a function of processing time at 110°C in days.

**Figure 4** illustrates a series of ghatti gum dispersions (15% w/w) for control and selected processed samples from Table 1 for which images were taken 30 minutes after inverting a universal container into which the samples were places.

**Figure 5** illustrates a graph of dynamic viscosity values (n') plotted as a function of oscillation frequency for karaya gum, a control sample of ghatti gum (lot 40138) and modified ghatti gum according to the present invention (heated for 168 hours at 110°C).

Experimental - gum modification

(1) *Initial modification studies*

Commercial ghatti gum (Gums & Colloids India, regular gum ghatti Lot 40138) was used. Dust, bark and particulate matter associated with the gum nodules were removed manually from the samples and the nodules were kibbled (1-2mm). Controlled heating of the kibbled material was carried out at 110°C, at atmospheric pressure, for various times using a Catterson Smith convection oven. Heating the gum in the dry state results in increasing the weight average molecular weight and viscoelasticity. Processing of the gum using the standard conditions outlined above was found to also change the hydration capacity and increase the gel content of the gum. The results are provided in Table 1 and each parameter described in turn together with the methodology used to determine the characteristics of the new materials.

**Table 1.** Values obtained for the % loss on drying, gel fraction as a % and the solubility of treated gum ghatti (Lot No.40138) as a function of time.

| Heating time (days) | % loss on drying | % gel | % solubility / hydration |
| --- | --- | --- | --- |
| 0 | 12.2 | 2.1 | 97.9 |
| 1 | 1.0 | 20.6 | 79.4 |
| 2 | 1.4 | 31.0 | 69.0 |
| 3 | 1.2 | 46.5 | 53.5 |
| 7 | 1.4 | 54.0 | 46.0 |
| 10 | 1.8 | 66.1 | 33.8 |
| 17 | 1.8 | 71.3 | 28.6 |
| 21 | 2.0 | 73.0 | 29.1 |
| 28 | 0.5 | 74.1 | 27.4 |
| 35 | 0.6 | 74.0 | 27.5 |
| 49 | 0.7 | 75.2 | 26.0 |
| 56 | 0.9 | 80.1 | 21.1 |

Experimental - gum properties

A) Percentage loss on drying

The percentage loss on drying of the samples of gum was determined as follows: around 1.0g of each sample was weighed in duplicate into pre-weighed evaporation dishes and placed in an oven SANYO convection oven MOV-212F at 105°C, for 16 hours. After heating, the samples were taken out of the oven and cooled in a desiccator. The respective weights of each sample were then recorded. The dry matter was calculated from the loss of weight after heating.
It was observed that processing for just 1 day resulted in drastically reducing the percentage (%) loss on drying from 12 - 1%. Further processing for up to eight weeks was found to not greatly change the % loss on drying.

B) Changes in viscoelastic parameters

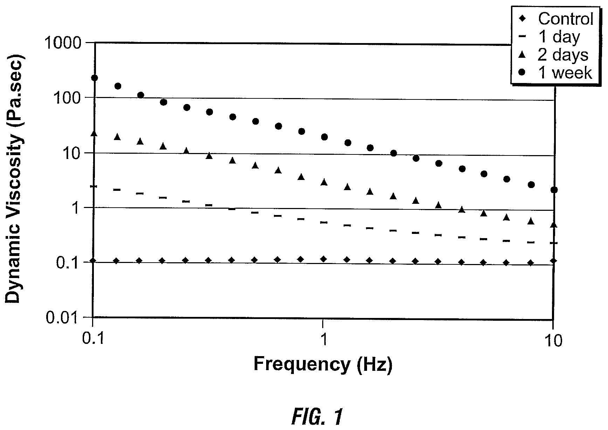

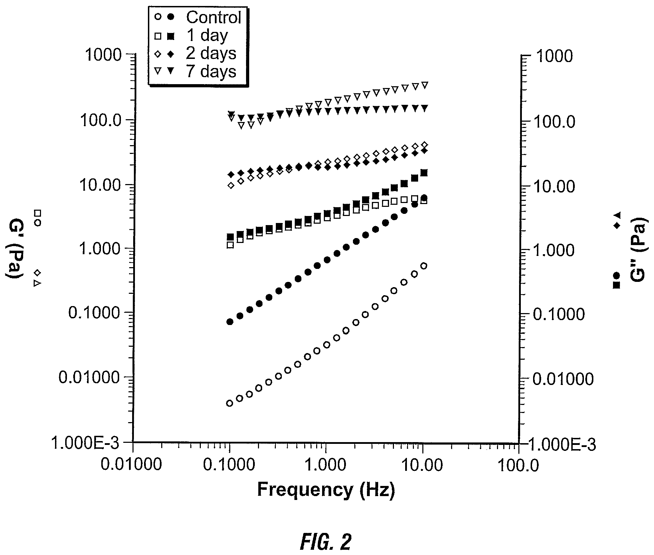

The viscoelastic parameters namely the storage modulus (G'), viscous modulus (G") and dynamic viscosity of a control sample and selected samples from Table 1 were determined using known controlled stress rheometer conditions at 10% w/w dispersions (based on dry solid content).
The changes recorded for the dynamic viscosity, storage modulus (G') and viscous modulus (G") are given in Figures 1 and 2 respectively. The results provided in Figure 1 show an increase in viscosity with increasing processing time. The results illustrate that gum ghatti can be produced with increased viscosity values and an increase to 200 Pa.s can be achieved from the starting value of around 0.1 Pa.s of control ghatti with 2% insoluble (gel) materials.
The control sample showed typical diluted solution behaviour where G" is greater than G' over the entire range of the frequency studied (Figure 2). The modified samples of gum ghatti had increased G' and G" and showed concentrated solution behaviour where there is a cross over of G' and G" for samples after processing for 24 and 48 hours. Gel-like systems can also be produced where G' and G" are almost independent of oscillation frequency over the entire range (Figure 2).

The results given above demonstrate that the viscosity and viscoelasticity of gum ghatti can be controlled by careful selection of the processing time.

C) Variation in solubility and gel content

The ghatti samples, listed in Table 1, were subjected to the following procedure: A 70 mm glass fibre paper (Fisher Scientific, 0.7 $\mu$m pore size) was dried in an oven (SANYO convection oven MOV-212F) at 105°C for 1 hour, transferred to a desiccator containing silica gel, left to cool and weighed (W1). About 0.1 g of the test material (S) was weighed in a plastic universal container (30 ml capacity) and 20 ml of distilled water was added and left to hydrate overnight on a roller mixer. The solution was filtered through the glass fibre paper under vacuum. Water, 10-20ml was then used to rinse the universal container and remove any residual insoluble material. The filter paper was dried at 105°C for 1 hour (or longer if a result showed that there was still water present), and transferred to a desiccator containing silica gel, left to cool and then the paper weighed (W2). The total insoluble (gel fraction) was calculated as follows wherein S is the weight of the test material:

$$\text{Total insoluble (\%)} = \frac{(W2-W1)}{S \times 100} \qquad \text{Formula (1)}$$

It is to be noted that the materials retained on the filter appeared to be swollen particles of gum of dimension greater than 0.7$\mu$m and are referred here to as gel.

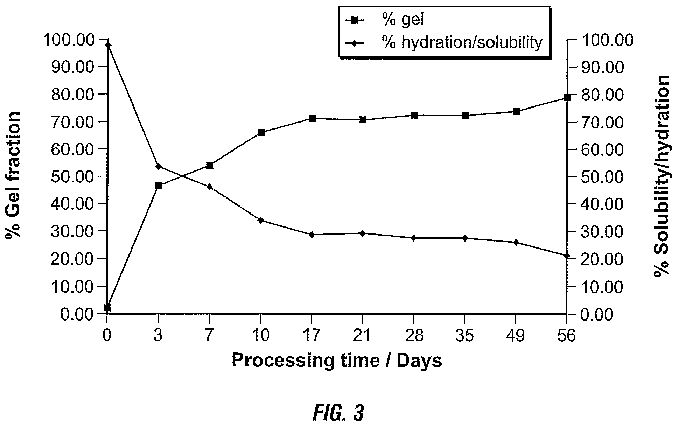

The results given in Table 1 are also shown in Figure 3, demonstrate that the % gel fraction and the degree of solubility can be controlled. The maximum gel % is achieved after processing for 21 days and further processing for up to 8 weeks does not greatly increase the maximum yield. However processing for up to 8 weeks can result in an 80% gel fraction.

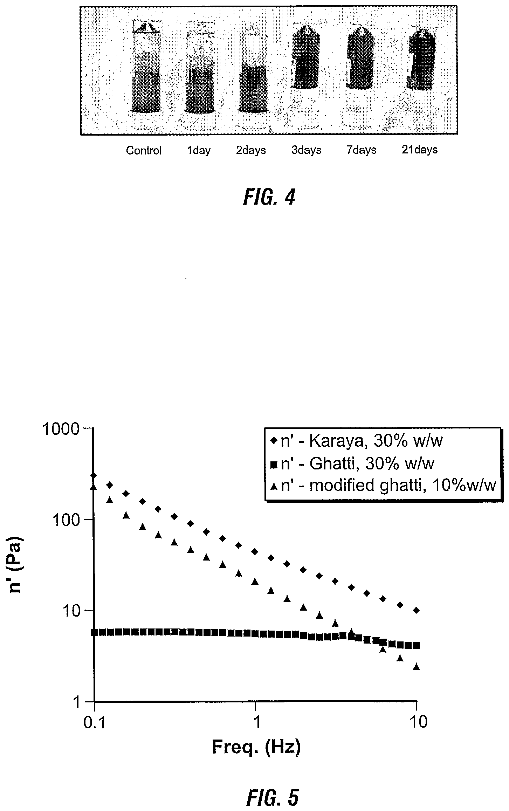

A further demonstration of the increase in the viscoelasticity and adhesion of the modified gum ghatti samples is given in Figure 4. Figure 4 shows 15% w/w (based on dry weight) dispersions of selected samples listed in Table 1 contained in a universal container. Following overnight hydration, the containers were inverted and all image taken after 30 minutes. The images showed that processed gum ghatti samples (3days and 7 days) do not flow and adhere increasingly to the surface of the container. The sample control showed the best flowability, and followed by the processed samples subjected to increasing processing time. With increasing processing time flowability decreased and adhesion increased.

D) Changes in water binding (swelling) capacity

Each sample of modified gum ghatti (0.1gm (based on solid dry content) was weighed and 0.5ml of water added. The water was immediately absorbed by the modified gum sample (21 days) whereas the water associated with the control sample remained fluid. After 30 seconds, the water was completely retained by the processed gum sample and it was possible to invert the weighing boat without losing any material. The same procedure was repeated but adding 1ml instead of 0.5ml. After 2 minutes, it was possible to invert the modified sample with very little flow of sample. After another 30 seconds the modified sample did not flow at all.

The water associated with the control sample during this time showed large water droplets and did not show any water binding capacity but rather dissolved the gum with time. The results are shown in Table 1, and demonstrate that the solubility and water absorption can be controlled.

It is therefore demonstrated above that the water solubility and absorption of the water by the gum can be changed as desired.

E) Producing physical properties in gum ghatti which allow specific characteristics of other hydrocolloids to be duplicated

Gum ghatti in the insoluble (gel) new material form according to a first aspect of the present invention can duplicate the functionality of other hydrocolloids, notably gum karaya, which has a wide range applicability in for example drug delivery and adhesion. Gum karaya, due to its high viscoelasticity has applications as a denture adhesive. The same characteristics of gum karaya have therefore been targeted in the present invention which give this gum its excellent water absorption capability and enable its use in food products such as mayonnaise and salad dressing, and in medical and pharmaceutical applications such as an adhesive agent for colostomy bags.

For the matching controlled experiments we have used a Karaya gum sample supplied by the Gums and Colloids Group (India) as white cleaned nodules. A sample was prepared by grinding into a powder and the proportion of insoluble (gel) fraction was determined using the method already described above. The insoluble (gel) proportion was 81%, which can be duplicated by the ghatti product obtained after modification (see Table 1).

The evidence of success of the present invention in duplicating the viscoelastic properties of gum karaya is given in Figure 5. This shows a plot of the dynamic viscosity of karaya gum at 30% compared with control ghatti at 30% concentration and the new ghatti gum produced by our invention at 10% w/w concentration. The results show that the new gum ghatti can match these characteristics of karaya and can do this at a much lower concentration. This invention similarly allows the modified gum ghatti to match the viscoelasticity characteristics of many other hydrocolloids used in pharmaceutical and cosmetics applications.

Modifying ghatti results in higher dynamic viscosity measurements at lower concentrations compared to standard ghatti measured at higher concentrations. These characteristics of the modified ghatti are also comparable to karaya at higher concentrations.

F) Adhesion to surface

A series of experiments was undertaken to demonstrate the stronger adhesion characteristics of the modified gum ghatti produced by according to the present invention. The gum produced after heating up to 72 hours and preferably up to 168 hours at 110°C in the solid state and preferentially for periods up to 8 weeks at 110°C was compared with a natural gum ghatti exudate which had not been subjected to the process described in this invention.

The modified sample of gum ghatti was found to be have stronger adhesion for up to 72 hours processing, and preferably up to 168 hours and more preferably up to 8 weeks processing when compared to natural gum ghatti exudate on the following surfaces: paper to paper adhesion; tackiness and sticking to skin and membrane of the human lip/mouth; adhesion to glass surfaces which stick the gum such that it will not fall when inverted and shaken; and, adhesion to plastic surfaces so that it adheres and will not fall off when inverted and shaken.

2) Extended studies

Further gum samples

For more extended studies of the behaviour of the modified gums in compositions such as denture adhesives, 5 gums were selected, along with two blends, as detailed in Table 2 along with the physical properties of these gums.

Denture adhesive formulation(s)

Denture adhesive examples 1 to 7 were made using the gums detailed in Table 2, as shown in Table 3

Table 2

| Description | Short description | Hydrogel | Mwx10$^5$ | 30% w/w aq. | | Moisture (%) |
|---|---|---|---|---|---|---|
| | | | | G' @ 1Hz | Visc @ 1s$^{-1}$ | |
| Control | Natural gum supplied as commercial product | 2% | 9.67 | 12.58 | 6.5 | 12.7 |
| High viscosity | Natural gum, obtained from certain areas in India. | 46% | 55.15 | 1166 | 658 | 4.9 |
| Shimla 1W | Produced by heat treatment of the base material (control ghatti) | 65% | 11.68 | 805.6 | 998 | 0.70 |
| Shimla 2W | Produced by heat treatment of the base material (control ghatti) | 80% | 5.73 | 1448 | 730 | 0.74 |
| Gum Karaya | Natural gum exudate. Supplied commercially | 80% | >100 | 1539 | 839 | 17.3 |

(continued)

| Description | Short description | Hydrogel | Mwx10[5] | 30% w/w aq. | | Moisture (%) |
|---|---|---|---|---|---|---|
| | | | | G' @ 1Hz | Visc @ 1s[-1] | |
| Blend 1 80% Control 20% High vise | Blend made up by mixing 80% of control gum ghatti and 20% high viscosity ghatti. | 11% | | 18.96 | 14.3 | 11.1(2) |
| Blend 2 20% Control 50% High viscosity 30% Shimla 1W | Blend made up by mixing 20% of control gum ghatti and 50% high viscosity ghatti and 30% Shimla 1W. | 41% | | 403 | 184 | 5.2(2) |

## Table 3

| | 1 | 2 | 3 | 4 | 5 | 6 | 7 | 8 |
|---|---|---|---|---|---|---|---|---|
| Flavour and colour | 4.1 | 4.1 | 4.1 | 4.1 | 4.1 | 4.1 | 4.1 | 4.1 |
| Petrolatum (*1) | 14 | 14 | 14 | 14 | 14 | 14 | 14 | 14 |
| Sunflower Oil (*2) | 27.9 | 27.9 | 27.9 | 27.9 | 27.9 | 27.9 | 27.9 | 27.9 |
| Cellulose Gum (Blanose 7H3SXF) (*3) | 33 | 33 | 33 | 33 | 33 | 33 | 33 | 33 |
| Gum Ghatti | 21 | | | | | | | |
| Gum Ghatti (Control) | | 21 | | | | | | |
| Gum Ghatti (High Viscosity) | | | 21 | | | | | |
| Gum Ghatti (Shimla 1W) | | | | 21 | | | | |
| Gum Ghatti (Shimla 2W) | | | | | 21 | | | |
| Gum Ghatti "Blend 1" | | | | | | 21 | | |
| Gum Ghatti "Blend 2" | | | | | | | 21 | 21 |
| Gum Karaya | | | | | | | | 21 |
| Total | 100 | 100 | 100 | 100 | 100 | 100 | 100 | |

These denture adhesives were then tested for performance in comparison with two commercial products, Commercial product 1 and Commercial product 2, and the following properties were measured:

- Dynamic Vapour Sorption (DVS) - this is a measurement of the uptake/loss of mass of a sample depending on time and relative humidity at specific temperatures, and was measured here at 25 °C on a sample of mass 10 mg, with a measurement cycle comprising the steps of drying the sample at humidity 0%, increasing the relative humidity to 98% for 3 hours, measure the uptake of moisture and subsequent change in mass as a function of time (dm/dt), then dry the sample again at humidity 0%. The results are plotted as a graph, from which the area under the curve of dm/dt vs time is measured.
- 3 minute hold, assessed using a Texture analyser by measuring the separation force exerted by a gel between a circular PMMA top plate and a bottom plate after a 3 minute contact time, wherein the sample comprises 0.25g test material + 1.5 gm of deionised water or artificial saliva, and the plates are of diameter 6.5 cm.
- Long term hold measurement of the separation force of initially unhydrated denture fixative using a texture analyser with a circular PMMA plate of diameter 6.5 cm over a moistened semi-permeable membrane forming part of an artificial mouth or over soft rubber. Force separation measurements are taken at 15 second intervals over a 90 minute period as the fixative hydrates and the assessment is made qualitatively in comparison with standard commercial products .
- Dynamic viscosity at 10 s$^{-1}$ (measured according to industry standard using a controlled stress rheometer).
- G' and G" values (measured as detailed above using an oscillation frequency sweep within LVR region using a controlled stress rheometer).

From the results in Tables 4 and 5 below it can be seen that samples incorporating the modified gums singly or as blends showed at least comparable performance as denture adhesives to the commercial products tested.

Table 4

| Test | Commercial product 1 | Commercial product 2 |
|---|---|---|
| DVS | Fast and short initial loss of water, followed by large intake of water that is lost very fast in a dry environment | As product 1, but the final loss of water is slower |
| 3 min hold | Within range | Within range |
| Long term hold | Acceptable values from the beginning | Acceptable values from beginning; Values increases after ~100' |
| Viscosity @10s$^{-1}$ unhydrated | Acceptable | Acceptable |
| Oscillation | Acceptable G', G" and n* | Acceptable G', G" and n* Low G" |

Without wishing to be bound by theory, it is believed that there is a direct link between the strength of the adhesive (as assessed by the 3 minutes hold and the long term hold), the DVS area, elastic modulus G', viscous modulus G", dynamic viscosity and viscosity. The modified gums present serve to increase the long term hold of the adhesive by imparting the correct properties of elasticity and viscosity on the mixture through slow hydration, and that this is only possible through being able to carefully control the properties of the gums through the presently claimed modification. The gums used here also surprisingly interact in a synergistic manner with other components, such as further adhesive substances (e.g. cellulose based products such as carboxymethyl-cellulose) and bases for creams, gels and ointments such as paraffin oil or petrolatum.

Table 5

| Mix | Control Ghatti + CMC | Blend 1 + CMC | High Viscosity + CMC | Karaya + CMC |
|---|---|---|---|---|
| DVS | High initial water content and long time to lose it. Hydration and dehydration profile similar to commercial product 1 | Lower hydration and dehydration profile than commercial product 1 | Lower hydration and dehydration profile than commercial product 1 | Lower hydration and dehydration profile than commercial product 1 |
| 3 min hold | Within range | Within range | Within range | Within range |

(continued)

| Mix | Control Ghatti + CMC | Blend 1 + CMC | High Viscosity + CMC | Karaya + CMC |
|---|---|---|---|---|
| pH | Slightly high | Slightly high | High | Within range |
| Long term hold | Very low values to start with Acceptable values after 20' High values after 30' | Values increase with time. Acceptable values after 20' | Low values to start with Acceptable values after 20' High values after 30' | Low values to start with Acceptable values after 20' High values after 30' |
| Viscosity @10s$^{-1}$ unhydrated | Low | Acceptable | Acceptable | Acceptable |
| Oscillation | High G' and $\eta$* Acceptable G" | High G' and $\eta$* Acceptable G" | High G' and $\eta$* Acceptable G" | High G' and $\eta$* Acceptable G" |

**Claims**

1. A process for the manufacture of a modified form of a polysaccharide gum which comprises the step of heating the natural gum, in the solid state, at atmospheric pressure at a temperature of between 100 to 130°C for at least 12 hours, wherein the modified gum has a gel fraction of between 10 and 80 wt% and a hydration value of between 60 and 10 wt%.

2. A process according to claim 1 which comprises heating for at least 24 hours, more preferably at least 3 days, further preferably at least 4 days, most preferably at least 6 days, especially at least 7 days, especially at least 14 days.

3. A process according to claim 1 or 2, wherein the gel fraction of the modified gum is > 20 wt%, most preferably > 30 wt%.

4. A process according to any of the preceding claims, wherein the hydration value is < 40 wt%, especially < 20 wt%.

5. A process according to any of the preceding claims, wherein when the modified gum is suspended or dissolved as a 10wt% dispersion in water, has a dynamic viscosity of at least 10 Pa.s at an oscillation frequency of 0.1 Hz.

6. A process according to any of the preceding claims, wherein the polysaccharide gum is, or is derived from, one or more of natural karaya, ghatti or kerensis gums.

7. Use of a modified polysaccharide gum produced according to any of the preceding claims in a denture adhesive.

**Patentansprüche**

1. Verfahren zum Herstellen einer modifizierten Form eines Polysaccharid-Gummis, umfassend den Schritt des Erhitzens des Naturgummis im festen Zustand auf atmosphärischen Druck bei einer Temperatur zwischen 100 und 130 °C über mindestens 12 Stunden,
wobei der modifizierte Gummi einen Gelanteil zwischen 10 und 80 Gew.-% und einen Hydratationswert zwischen 60 und 10 Gew.-% aufweist.

2. Verfahren nach Anspruch 1, umfassend das Erhitzen über mindestens 24 Stunden, bevorzugter mindestens 3 Tage, weiter bevorzugt mindestens 4 Tage, am meisten bevorzugt mindestens 6 Tage, insbesondere mindestens 7 Tage, insbesondere mindestens 14 Tage.

3. Verfahren nach Anspruch 1 oder 2, wobei der Gelanteil des modifizierten Gummis > 20 Gew.-%, am meisten bevorzugt > 30 Gew.-% beträgt.

4. Verfahren nach einem beliebigen der vorhergehenden Ansprüche, wobei der Hydratationswert < 40 Gew.-%, insbesondere < 20 Gew.-% beträgt.

**5.** Verfahren nach einem beliebigen der vorhergehenden Ansprüche, wobei, wenn der modifizierte Gummi als eine Dispersion mit 10 Gew.-% in Wasser suspendiert oder gelöst wird, eine dynamische Viskosität von mindestens 10 Pa.s bei einer Oszillationsfrequenz von 0,1 Hz aufweist.

**6.** Verfahren nach einem beliebigen der vorhergehenden Ansprüche, wobei das Polysaccharid-Gummi eines oder mehrere von natürlichem Karaya, Ghatti oder Kerensis-Gummi ist oder davon abgeleitet ist.

**7.** Verwendung eines nach einem beliebigen der vorhergehenden Ansprüche hergestellten modifizierten Polysaccharid-Gummis in einem Prothesenklebstoff.

**Revendications**

**1.** Procédé de fabrication d'une forme modifiée d'une gomme polysaccharidique qui comprend l'étape consistant à chauffer la gomme naturelle, à l'état solide, à la pression atmosphérique à une température comprise entre 100 et 130 °C pendant au moins 12 heures,
dans lequel la gomme modifiée a une fraction de gel comprise entre 10 et 80 % en poids et une valeur d'hydratation comprise entre 60 et 10 % en poids.

**2.** Procédé selon la revendication 1, qui comprend le chauffage pendant au moins 24 heures, plus préférentiellement au moins 3 jours, encore plus préférentiellement au moins 4 jours, de manière préférée entre toutes au moins 6 jours, en particulier au moins 7 jours, en particulier au moins 14 jours.

**3.** Procédé selon la revendication 1 ou 2, dans lequel la fraction de gel de la gomme modifiée est > 20 % en poids, de manière préférée entre toutes > 30 % en poids.

**4.** Procédé selon l'une quelconque des revendications précédentes, dans lequel la valeur d'hydratation est < 40 % en poids, en particulier < 20 % en poids.

**5.** Procédé selon l'une quelconque des revendications précédentes, dans lequel, lorsque la gomme modifiée est en suspension ou dissoute sous forme d'une dispersion dans l'eau à 10 % en poids, elle présente une viscosité dynamique d'au moins 10 Pa.s à une fréquence d'oscillation de 0,1 Hz.

**6.** Procédé selon l'une quelconque des revendications précédentes, dans lequel la gomme polysaccharidique est, ou est dérivée de, une ou plusieurs des gommes naturelles karaya, ghatti ou arabique de kerensis.

**7.** Utilisation d'une gomme polysaccharidique modifiée produite selon l'une quelconque des revendications précédentes dans un adhésif pour prothèse dentaire.

*FIG. 1*

*FIG. 2*

*FIG. 3*

Control    1day    2days    3days    7days    21days

*FIG. 4*

*FIG. 5*

**EP 2 180 872 B1**

**REFERENCES CITED IN THE DESCRIPTION**

**Patent documents cited in the description**

- US 3833518 A
- DE 1909209 A
- US 4356819 A
- US 4474902 A